# EUROPÄISCHE PATENTSCHRIFT

(11) **EP 1 358 149 B1**
(45) Veröffentlichungstag und Bekanntmachung des Hinweises auf die Patenterteilung: **30.05.2007**
(21) Anmeldenummer: 02710816.6
(22) Anmeldetag: 29.01.2002
(51) Int. Cl.: C07C 51/487, B01J 21/18, B01J 23/44, B01J 35/10, B01J 35/06

(54) **VERFAHREN ZUR REINIGUNG VON ROH-TEREPHTHALSÄURE UND DAFÜR GEEIGNETE KOHLENSTOFFFASERN ENTHALTENDE KATALYSATOREN**
METHOD FOR CLEANING CRUDE TEREPHTHALIC ACID AND CATALYSTS SUITABLE FOR THE SAME AND CONTAINING CARBON FIBRES
PROCEDE POUR NETTOYER L'ACIDE TEREPHTALIQUE BRUT ET CATALYSEURS ADAPTES A CET EFFET, CONTENANT DES FIBRES DE CARBONE

(30) Priorität: 31.01.2001 DE 10104224; 27.08.2001 DE 10141848
(43) Veröffentlichungstag der Anmeldung: 05.11.2003
(73) Patentinhaber: BASF Aktiengesellschaft, 67056 Ludwigshafen (DE)
(72) Erfinder: HAAKE, Mathias, 68161 Mannheim (DE); SCHWAB, Ekkehard, 67434 Neustadt (DE); KOCH, Michael, 67346 Speyer (DE); MÜLLER, Hans-Joachim, 82319 Starnberg (DE); STROEZEL, Manfred, 68549 Ilvesheim (DE); PETERSEN, Hermann, 67269 Grünstadt (DE); SCHREYER, Peter, 69469 Weinheim (DE)
(74) Vertreter: Isenbruck, Günter
(86) Internationale Anmeldenummer: PCT/EP2002/000900
(87) Internationale Veröffentlichungsnummer: WO 2002/060851

(56) Entgegenhaltungen:
- EP-A- 0 418 682
- DE-A- 3 229 905
- GB-A- 994 769

## Beschreibung

Die Erfindung betrifft Verfahren zur Reinigung von Roh-Terephthalsäure durch katalytische hydrierende Nachbehandlung an einem Katalysatormaterial, das mindestens ein auf Kohlenstoffträger aufgebrachtes Hydriermetall enthält. Die Erfindung betrifft ferner einen Reaktor und Kohlenstofffasern enthaltende Katalysatoren, Verfahren zu ihrer Herstellung und ihre Verwendung insbesondere als Hydrierkatalysator.

Die überwiegende Menge an Terephthalsäure wird technisch nach dem Amoco-Flüssigphasen-Oxidationsverfahren hergestellt. In diesem Verfahren wird p-Xylol in 95 %-iger Essigsäure mit Hilfe eines aus Co- und Mn-Salzen sowie Bromverbindungen bestehenden Katalysatorsystems mit Luftsauerstoff unter Druck oxidiert. Im Anschluss an die Oxidationsstufe ist beim Amoco-Verfahren eine Reinigungsstufe erforderlich, wenn die erzeugte Terephthalsäure zu Fasern weiterverarbeitet werden soll. Die Aufgabe dieser Reinigungsstufe ist es im wesentlichen, den durch nur partielle Oxidation gebildeten 4-Carboxybenzaldehyd in eine nicht störende bzw. leicht abzutrennende Verbindung zu überführen. Typischerweise entstehen etwa 5000 ppm 4-Carboxybenzaldehyd. Diese Verbindung muss entfernt werden, weil dadurch die Polykondensationsreaktion bei der Weiterverarbeitung der Terephthalsäure gestört wird. Andererseits entstehen auch störende Gelbfärbungen im Kondensationsprodukt.

Am weitesten verbreitet ist zur Lösung dieser Aufgabe eine nachgeschaltete Hydrierstufe, in der eine wässrige Lösung der Roh-Terephthalsäure bei Temperaturen um 250°C unter Druck an einem Edelmetall-Kohlen-Kontakt behandelt wird. Dabei erfolgt die Umsetzung des 4-Carboxybenzaldehyds zur p-Tolylsäure, die im Gegensatz zum Aldehyd durch Kristallisation leicht vom Wertprodukt Terephthalsäure abgetrennt werden kann. Das Grundprinzip dieses Verfahrens ist in der US 3 584 039 (1967) beschrieben. Die im Katalysator eingesetzten Kohlenstoffträger sind pulver- oder granulatförmig.

Bei der technischen Ausführung des Hydrier-Verfahrens sind stückige Katalysatoren gebräuchlich, die typischerweise 0,5 Gew.-% Palladium auf einem technisch verfügbaren Kohlenstoffträger enthalten, vergleiche EP-A-0 879 641. Obwohl die Hydrieraufgabe damit befriedigend gelöst werden kann, besitzen die eingesetzten Katalysatoren in der praktischen Anwendung einige Nachteile. Insbesondere weisen Katalysatorbetten aus stückigen Katalysatoren, die auf Kohlenstoff als Träger aufbauen, den Nachteil auf, dass auf Grund der relativ geringen mechanischen Stabilität des Trägermaterials bei unvermeidlich unter Betriebsbedingungen auftretenden Bewegungen im Katalysatorbett Abrieb entsteht, der im weiteren Verlauf vom Produkt abgetrennt werden muss. Zudem ist mit diesem Abrieb auch ein Verlust der teuren Edelmetall-Aktivkomponente verbunden. Darüber hinaus wird häufig beobachtet, dass es auch auf anderem Wege im Laufe der Betriebszeit zu Verlusten der Edelmetall-Aktivkomponente kommt, wenn diese nicht in ausreichendem Maße chemisch auf dem Trägermaterial fixiert wurde.

Festbett-Katalysatoren, die aktivierte Kohlenstofffasern enthalten, auf denen eine oder mehrere aktive Katalysator-Komponenten niedergeschlagen sind, sind in der DE-A-32 29 905 beschrieben. Die Kohlenstofffasern sind in Form eines Gefüges ausgebildet, in dem sie miteinander verschlungen sind und einen volumenfüllenden Zustand bilden. Dabei liegen die Kohlenstofffasern z.B. in filzähnlicher Form vor.

Monolithische Katalysatoren werden zur Hydrierung seit längerer Zeit eingesetzt. In der EP-A-0 827 944 ist ein derartiges Verfahren zur Hydrierung beschrieben, bei dem der Katalysator als Katalysatorpackung in vorzugsweise monolithischer Form eingesetzt wird. Die Katalysatorpackung wird durch Aufbringung mindestens einer als Katalysator aktiven Substanz auf Gewebe, Gestricke oder Folien als Trägermaterial hergestellt.

Eine Beschichtung der Katalysatorträger mit Aktivmassen kann nach unterschiedlichen Verfahren erfolgen. In der EP-A-0 965 384 sind Tränkverfahren zur Aufbringung von Aktivmasse auf strukturierte Träger oder Monolithe beschrieben. Dabei wird ein Tränkmedium eingesetzt, das eine Oberflächenspannung von höchstens 50 mN/m hat. Aus den strukturierten Trägern werden Monolithe hergestellt. Die für den Träger verwendeten Werkstoffe können metallische oder keramische Werkstoffe oder Kunststoffe umfassen, wobei auch Kohlenstoff genannt ist. Wie auch in EP-A-0 827 944 beschrieben, werden bevorzugt Metallgewebestreifen als Katalysatorträger oder Katalysatoren eingesetzt.

In der WO 99/26721 ist die Herstellung eines Katalysatorträgers aus Aktivkohlefasern beschrieben. Dazu werden Kunstseidefasern in Aktivkohlefasern überführt und mit katalytisch aktiven Metallen imprägniert oder durch Kationenaustausch behandelt. Eine Formgebung des Katalysatorträgers in Geweben und anderen flächigen Substraten wird beschrieben. Der Katalysator wird in dieser Gewebeform eingesetzt.

Eine wichtige Anwendung von Hydrierkatalysatoren ist die Herstellung und Reinigung von Terephthalsäure.

Aufgabe der vorliegenden Erfindung ist die Bereitstellung eines Verfahrens zur Reinigung von Roh-Terephthalsäure und von Hydrierkatalysatoren, die die Nachteile der bekannten Katalysatoren vermeiden. Die Katalysatoren sollen insbesondere eine erhöhte mechanische Stabilität und Abriebfestigkeit aufweisen und vorteilhaft bei der hydrierenden Nachbehandlung von Roh-Terephthalsäure einsetzbar sein.

Die Aufgabe wird erfindungsgemäß gelöst durch ein Verfahren zur Reinigung von Roh-Terephthalsäure durch katalytische hydrierende Nachbehandlung an einem Katalysatormaterial, das mindestens ein auf Kohlenstoffträger aufgebrachtes Hydriermetall enthält, wobei als Kohlenstoffträger Kohlenstofffasern eingesetzt werden.

Die katalytische hydrierende Nachbehandlung kann dabei wie in US 3,584,039 beschrieben erfolgen. Insbesondere dient der Katalysator zur Hydrierung von 4-Carboxybenzaldehyd zu p-Tolylsäure. Besonders bevorzugt wird das Verfahren bei Temperaturen im Bereich von oberhalb 200°C und unter einem Druck von vorzugsweise 50 bis 100 bar durchgeführt. Dabei kann die hydrierende Nachbehandlung kontinuierlich oder diskontinuierlich durchgeführt werden.

Die Kohlenstofffasern können dabei in beliebiger geeigneter Form im Reaktor angeordnet sein. Sie können beispielsweise geordnet oder ungeordnet im Reaktor vorliegen, beispielsweise ungeordnet in Form eines Filzes, wie er in DE-A-32 29 905 beschrieben ist, oder geordnet in Form von flächigen Gebilden, wie sie beispielsweise in WO 99/26721 beschrieben sind. Es ist jede räumliche Anordnung der Kohlenstofffasem geeignet, die eine katalytische hydrierende Nachbehandlung von Roh-Terephthalsäure erlaubt. Sie muss daher den Kontakt von Roh-Terephthalsäure mit den Kohlenstofffasern und einen Stoffaustausch erlauben. Deshalb sind die Kohlenstofffasern in der Regel so angeordnet, dass die Roh-Terephthalsäure-Lösung bei der katalytischen Hydrierung an den Kohlenstofffasern entlang streichen kann.

Bei einer kontinuierlich betriebenen Flüssigfahrweise sind die Kohlenstofffasern daher vorzugsweise in einem Reaktor so angeordnet, dass der Flüssigkeitsstrom auf den Kohlenstofffasern durch den Reaktor läuft. Bevorzugte Geometrien sind nachstehend ausführlicher beschrieben.

Das Katalysatormaterial kann z.B. in (flächiger) Form von Geweben, Gestricken, Gewirken und/oder Filzen oder in Form paralleler Fasern oder Bänder vorliegen. Die parallelen Fasern oder Bänder können entlang der Durchströmungsrichtung eines Reaktors ausgerichtet sein.

Gemäß einer Ausführungsform der vorliegenden Erfindung weist das flächige Katalysatormaterial mindestens zwei einander gegenüberliegende Kanten auf, an denen das Katalysatormaterial in einem Reaktor formstabil befestigt wird. Dabei erstrecken sich vorzugsweise mehrere Streifen des flächigen Katalysatormaterials parallel zu einer Vorzugsrichtung im Reaktor, und sie sind räumlich so zueinander angeordnet, dass ein Abrieb an den Streifen durch gegenseitiges Berühren der Streifen untereinander oder mit den Reaktorwänden während des Reaktorbetriebs weitgehend oder vorzugsweise vollständig verhindert wird.

Der Ausdruck "einander gegenüberliegende Kanten" bezieht sich auf ein flächiges Katalysatormaterial, das zwei die Fläche an zwei Seiten begrenzende Kanten aufweist. Die Kanten sind dabei im wesentlichen parallel zueinander. Sie sind vorzugsweise gerade, können jedoch auch andere Formen wie eine Wellenform oder andere Linien zeigen. Sie sind vorzugsweise parallel zueinander, können jedoch auch Winkel von beispielsweise bis zu 20° zueinander bilden. Ein Parallelogramm weist beispielsweise jeweils zwei derartige parallele einander gegenüberliegende Kanten auf. Eine rechteckige oder quadratische Fläche weist zwei jeweils zueinander senkrecht stehende einander gegenüberliegende Kantenpaare auf, die die Fläche begrenzen. Die einander gegenüberliegenden Kanten sind erfindungsgemäß so ausgestaltet, dass sie eine Befestigung des flächigen Katalysatormaterials in einem Reaktor erlauben. Die Befestigung erfolgt dabei formstabil. Der Ausdruck "formstabil" bedeutet, dass das im Reaktor befestigte Katalysatormaterial vor, während und nach dem Betrieb des Reaktors eine flächige Form beibehält und nicht etwa zu einem Knäuel oder Haufen zusammengepresst wird. Beispielsweise kann das flächige Katalysatormaterial einem Rahsegel eines Segelschiffs vergleichbar im Reaktor befestigt sein. Die flächige Form erstreckt sich dabei durch den Reaktor und wird bei einem Betrieb des Reaktors im wesentlichen nicht verändert.

Es ist beispielsweise denkbar, dass in einem zylinderförmigen Reaktor im oberen und unteren Bereich des Reaktors senkrecht zur Reaktorlängsachse zwei Gitterroste angeordnet sind, zwischen denen das flächige Katalysatormaterial aufgespannt ist bzw. die Fasern oder Bänder aufgespannt sind. Wenn die zueinander parallelen Gitter eine Anzahl von Streben aufweisen, so können mehrere Streifen des flächigen Katalysatormaterials parallel zueinander an diesen Befestigungsvorrichtungen aufgespannt sein. Der Ausdruck "Streifen" bezeichnet dabei im wesentlichen rechteckige Flächen des flächigen Katalysatormaterials, die in dieser ebenen, flächigen Form im Reaktor formstabil befestigt, insbesondere aufgespannt sind. Mehrere Streifen des flächigen Katalysatormaterials liegen dabei vorzugsweise parallel zu einer Vorzugsrichtung im Reaktor vor (etwa vergleichbar mit der Vorzugsorientierung von Flüssigkristallen in einer nematischen Phase). Die Vorzugsrichtung kann beispielsweise bei einem rohrförmigen Reaktor entlang der Längsrichtung des Rohres verlaufen. Es können jedoch auch Winkel zur Längsrichtung des Reaktors eingenommen werden. Vorzugsweise verlaufen die Streifen in dem Reaktor so, dass ihre Vorzugsrichtung mit der Fließrichtung eines Reaktionsgemisches im wesentlichen übereinstimmt.

Die Streifen sind dabei vorzugsweise so im Reaktor und zueinander räumlich angeordnet, dass ein gegenseitiger Abrieb an den Streifen durch Berühren der Streifen untereinander oder mit den Reaktorwänden während des Reaktorbetriebs weitgehend verhindert wird. Dies wird durch ausreichende Abstände der Streifen zueinander und zu den Reaktorwänden sichergestellt. Geeignete Geometrien lassen sich durch einfache Versuche schnell ermitteln. Ein auftretender Abrieb lässt sich im Reaktoraustrag einfach optisch feststellen.

Variationen der vorstehend beispielhaft genannten Geometrien - beispielsweise nur im oberen Bereich fixierte (frei hängende) oder in einem Rahmen (teil)fixierte Katalysatorgewebe- sind erfindungsgemäß mit eingeschlossen.

Die Erfindung betrifft entsprechend auch einen Reaktor, enthaltend ein flächiges Katalysatormaterial in Form von Geweben, Gestricken, Gewirken und/oder Filzen, das mindestens ein auf Kohlenstofffasern aufgebrachtes Hydriermetall enthält und das mindestens zwei einander gegenüberliegende Kanten aufweist, an denen das Katalysatormaterial in Reaktorform stabil befestigt ist, oder enthaltend das Katalysatormaterial in Form paralleler Fasern oder Bänder. Der Reaktor mit dem darin enthaltenen flächigen Katalysatormaterial weist vorzugsweise die vorstehend angegebenen Geometrien auf.

Gemäß einer weiteren Ausführungsform der Erfindung wird ein im folgenden beschriebener monolithischer Katalysator eingesetzt. Die Beschreibung der Kohlenstofffasern und Hydriermetalle sowie Verfahren zur Herstellung gelten auch für die vorstehend beschriebene Ausführungsform.

Die Aufgabe wird gemäß einer weiteren Ausführungsform der vorliegenden Erfindung durch einen Katalysator gelöst, der mindestens ein auf Kohlenstoffträger aufgebrachtes Hydriermetall enthält, dadurch gekennzeichnet, dass als Kohlenstoffträger Kohlenstofffasern eingesetzt werden, wobei die Kohlenstoffträger eine BET-Oberfläche von < 500 m²/g, bevorzugt < 300 m²/g, besonders bevorzugt von < 100 m²/g, ganz besonders bevorzugt von < 50 m²/g und insbesondere von < 10m²/g aufweisen. Die untere Grenze der möglichen BET-Oberfläche der Kohlenstoffträger ist im allgemeinen die BET-Oberfläche der geometrischen Faseroberfläche, entsprechend einer Porösität von 0 %.

Diese Kohlenstofffasern zeichnen sich durch eine hohe mechanische Stabilität aus. Z.B. beträgt die Reißfestigkeit (tensile strength) dieser Kohlenstofffasern im allgemeinen bis zu ca. 60.000 bar, bevorzugt von ca. 13.000 bar bis 35.000 bar.

Geeignete Geometrien und weitere Eigenschaften der erfindungsgemäßen Katalysatoren wurden bereits vorstehend genannt. Geeignete Hydriermetalle werden im folgenden genannt.

Die Aufgabe wird weiterhin erfindungsgemäß gelöst durch einen monolithischen Katalysator, enthaltend mindestens ein Katalysatormaterial, das mindestens ein auf Kohlenstoffasern aufgebrachtes Hydriermetall enthält, und mindestens ein vom Katalysatormaterial unterschiedliches und mit ihm verbundenes Stütz- oder Skelettelement, das das Katalysatormaterial mechanisch abstützt und in der monolithischen Form hält.

Monolithische Strukturen sind beispielsweise in EP-A-0 564 830 beschrieben. Monolithische Strukturen unterscheiden sich von teilchenförmigen Katalysatoren oder deren Trägern dadurch, dass sie in wesentlich weniger Teilen vorliegen als die teilchenförmigen (pulverförmigen oder granulatförmigen) Katalysatoren. Für einen gegebenen Reaktor kann der Katalysator in Form eines einzigen Monolithen eingesetzt werden, oder mehrere Monolithen können zur Bildung des Festbettkatalysators gestapelt werden. Die Anzahl der Monolithen ist dabei jedoch gering, beispielsweise werden 1 bis 10 Monolithen für eine Katalysatorpackung verwendet. Die Monolithen weisen in der Regel ausgedehnte dreidimensionale Strukturen auf, die von durchgehenden Kanälen durchzogen sind. Dabei können die Monolithe beliebige geeignete äußerer Formen aufweisen, beispielsweise kubisch, quaderförmig, zylinderförmig usw. Die durchgehenden Kanäle können eine beliebige Geometrie aufweisen, beispielsweise können sie in einer Wabenstruktur wie in einem Abgaskatalysator vorliegen. Häufig werden Katalysatormonolithe durch Verformen von flächigen Trägerstrukturen hergestellt, beispielsweise durch Aufrollen oder Knicken der flächigen Strukturen zu dreidimensionalen Monolithen. Ausgehend von flächigen Substraten kann die äußere Form des Monolithen dabei einfach an gegebene Reaktorgeometrien angepasst werden.

Es wurde gefunden, dass die vorstehend beschriebenen Probleme in technisch einfacher und wirtschaftlich vorteilhafter Weise auch gelöst werden können, wenn als Katalysatorträger statt der im Stand der Technik gebräuchlichen stückigen Katalysatorträger monolithische Katalysatorpackungen in der Festbetthydrierung verwendet werden. Mit monolithischen Strukturen sind im Sinne der vorliegenden Erfindung insbesondere solche Strukturen gemeint, bei denen zunächst ein flächiger Kohlenstoffträger, beispielsweise ein Gewebe aus Kohlenstoff-Fasern, zunächst mit einem aktiven Hydriermetall beladen wird und das so erhaltene aktivierte Gewebe in einem zweiten Schritt zum gewünschten monolithischen Katalysator-Formkörper weiterverarbeitet wird.

Die erhaltenen monolithischen Strukturen zeichnen sich wie die vorstehenden im Reaktor angeordneten Streifen dadurch aus, dass eine kontrollierte Durchströmung des Katalysatorbetts möglich ist. Ein Aneinanderreiben der Katalysatorpartikel wird in jedem Fall vermieden. Auf Grund der geordneten Struktur des Katalysatorbetts ergeben sich verbesserte Möglichkeiten für den strömungstechnisch optimalen Betrieb des Katalysatorbetts mit verbessertem Stoffübergang zwischen den im Reaktor vorliegenden Phasen. Eine theoretische Behandlung dieses Optimierungspotentials findet sich beispielsweise in "Monoliths in Multiphase Catalytic Processes" (CatTech 3(1999), 24 ff). Die dort beschriebenen monolithischen Katalysatoren basieren sämtlich auf extrudierten Formkörpern, welche bei der Beladung mit aktiven Metallen größere präparative Schwierigkeiten bieten als die hier vorgeschlagenen Katalysatoren, welche vorzugsweise ausgehend von flächigen Vorstufen zugänglich sind. In der zitierten Veröffentlichung findet sich lediglich ein Hinweis darauf, dass die Herstellung monolithischer Strukturen auch ausgehend von dünnen Blechen möglich ist. Die Herstellung derartiger, von flächigen Vorstufen ausgehenden monolithischen Katalysatoren ist beispielsweise in der EP-A-0 564 830, EP-A-0 827 944 und EP-A-0 965 384 beschrieben. Für die dreidimensionale Gestaltung der erfindungsgemäßen Katalysatoren kann auch auf diese Schriften verwiesen werden.

Die erfindungsgemäßen monolithischen, auf Kohlenstoff-Fasermaterialien basierenden Katalysatoren eignen sich neben der Ablösung von konventionellen Katalysatorfestbetten insbesondere auch zur Substitution von Katalysatoren, die in suspendierter Form auf Kohlenstoff-Trägern eingesetzt werden. Derartige Katalysatoren finden vor allem in zahlreichen Hydrierverfahren insbesondere im Bereich der Feinchemie Verwendung. Bei diesen, meistens diskontinuierlich betriebenen Verfahren, muss der Katalysator nach Beendigung der Reaktion vom Reaktionsgemisch abgetrennt werden. Dies erfolgt entweder durch Sedimentation oder aber durch Filtration. Die erfindungsgemäßen Katalysatoren zeichnen sich dadurch aus, daß sie bei gleicher Hydrieraktivität ohne die üblichen mehr oder weniger aufwendigen Operationen einfach aus dem Reaktionsgemisch entnommen werden können. Es ergibt sich dadurch eine Verkürzung der Chargenzeiten und eine Verbesserung der Wirtschaftlichkeit der Verfahren. In vielen Fällen kann der zuvor für die Suspensionshydrierung verwendete Reaktor mit kleinen technischen Modifikationen zur Aufnahme des monolithischen Katalysatorblocks weiterverwendet werden.

Der erfindungsgemäße monolithische Katalysator weist eine Kombination aus einem Katalysatormaterial und einem Skelett- oder Stützelement auf.

Das mindestens eine Stütz- oder Skelettelement dient dazu, eine stabile und permanente Formgebung des auf Kohlenstofffasern basierenden Katalysatormaterials zu erlauben. Im Katalysatormaterial liegen dabei die Kohlenstofffasern vorzugsweise flächig in Form von Geweben, Gestricken, Wirken und/oder Filzen vor. Besonders bevorzugt liegen sie in Form von Geweben oder Gestricken, insbesondere in Form von Geweben vor. Geeignete Kohlenstofffasern sind beispielsweise in DE-A-32 29 905, WO 99/26721 und Ullmann's Encyclopedia Industrial Chemistry, Section: Fibers, Synthetic Inorganic, Composite Materials Carbon Fibers beschrieben. Es können alle geeigneten Kohlenstofffasern eingesetzt werden. Derartige Fasern sind nach dem Stand der Technik beispielsweise aus Polyestern, Polyamiden, Polyolefinen und dergleichen zugänglich. Erfindungsgemäß besonders bevorzugt sind Fasern, Gewebe, Gewirke, Gestricke oder Filze mit folgenden Eigenschaften: spezifische Dichte von 80 bis 600 g/m², Fadendichte von 3 bis 15 Fäden/cm, Fadendurchmesser von 0,1 bis 0,9 mm. Besonders bevorzugt werden hochreißfeste Faserbündel eingesetzt. Die BET-Oberflächen betragen vorzugsweise weniger als 300 m²/g, besonders bevorzugt weniger als 100 m²/g, insbesondere weniger als 15 m²/g. Die Porosität ist vorzugsweise kleiner als 0,5 ml/g. Derartige Fasern werden beispielsweise von Tenax Fibers vertrieben. Geeignete Fasern sind unter textileworld.com im Internet beschrieben.

Das Katalysatormaterial ist mit dem Stütz- oder Skelettelement verbunden. Das Stütz- oder Skelettelement stützt das Katalysatormaterial mechanisch ab und hält es in der monolithischen Form. Insbesondere die Kohlenstofffasergewebe weisen häufig nicht für eine Festbettanwendung ausreichende mechanische Eigenschaften wie Härte und Formstabilität auf. Sie werden deshalb erfindungsgemäß mit einem oder mehreren Stütz- oder Skelettelementen verbunden, wodurch das Katalysatormaterial mechanisch stabilisiert und in der gewünschten Monolithform gehalten wird. Dadurch werden Deformationen durch Erschütterungen und beim Durchfluss von Reaktanden vermieden. Das Stütz- oder Skelettelement kann dabei jede beliebige für diese Anwendung geeignete Form aufweisen. Die Verbindung mit dem Katalysatormaterial kann dabei ebenfalls auf beliebige geeignete Weise erfolgen, beispielsweise durch Verhaken, Verkleben usw. oder auch gemeinsames Verstricken oder Verweben von Fasern aus Kohlenstoff und Stütz- oder Skelettelement.

Vorzugsweise liegt das mindestens eine Stütz- oder Skelettelement flächig in Form von Geweben, Gestricken, Gewirken, Filzen und/oder Lochblechen vor. Dabei können diese flächigen Formen weiterhin gewellt oder gefalzt sein. Besonders bevorzugt bilden die jeweils flächigen Stütz- oder Skelettelemente und das Katalysatormaterial alternierende Schichten im monolithischen Katalysator. Das Stütz- oder Skelettelement kann dabei aus beliebigen geeigneten Werkstoffen aufgebaut sein, beispielsweise aus Metallen und deren Legierungen, Kunststoffen oder Keramiken. Für verwendbare Materialien kann auf EP-A-0 965 384 verwiesen werden, siehe die Beschreibung der Katalysatorträger. Besonders bevorzugt liegt das Stütz- oder Skelettelement aus individuell chemisch beständigen Metallen vor.

Gemäß einer Ausführungsform der Erfindung liegen sowohl das Katalysatormaterial als auch das mindestens eine Stütz- oder Skelettelement bevorzugt in Gewebeform als flächige Schichtung vor, die in einen zylindrischen Monolithen mit mehreren parallel zur Längsachse des Zylinders durchströmbaren Kanälen verformt ist.

Das Katalysatormaterial enthält mindestens ein auf den beschriebenen Kohlenstofffasern aufgebrachtes Hydriermetall. Als Hydriermetalle können dabei alle Metalle eingesetzt werden, die die Hydrierung organischer Verbindungen katalysieren. Vorzugsweise handelt es sich um Metalle der Gruppen VIII oder IB des Periodensystems der Elemente. Die Hydriermetalle werden in üblichen Mengen nach bekannten Verfahren auf die Kohlenstofffasem aufgebracht. Geeignete Tränkverfahren zur Aufbringung der Aktivmasse sind in EP-A-0 965 384 beschrieben. Auch andere Verfahren zur Aufbringung und Fixierung der Katalysatormetalle auf den Kohlenstofffasern können angewendet werden. Die Kohlenstofffasern können beispielsweise durch Oxidation von Kunststofffasern erhalten werden, wobei die Aktivmetalle bereits auf die Kunststofffasern aufgebracht oder in sie eingebracht sein können. Sie können aber auch nachträglich aufgebracht werden. Beispielsweise kann die Herstellung des monolithischen Katalysators auch zunächst unter Verwendung von Kunststofffasern in geeigneter Form, beispielsweise Gewebeform, erfolgen, wobei nachfolgend das Kunststoffmaterial zu Kohlenstofffasern bzw. deren Geweben oxidiert wird.

Die Erfindung betrifft auch ein Verfahren zur Herstellung des monolithischen Katalysators durch separate Herstellung des Katalysatormaterials und mindestens einen Stütz- oder Skelettelementes, Zusammenfügen dieser und Verformen zu einem Monolithen.

Die monolithischen Katalysatoren wie auch die vorstehend beschriebenen Reaktoren können allgemein zur Hydrierung von ungesättigten organischen Verbindungen eingesetzt werden. Sie können auch zur Selektivhydrierung von Kohlenstoff-Kohlenstoff-Doppel- oder -Dreifachbindungen und/oder hydrierbaren funktionellen Gruppen in diese enthaltenen organischen Verbindungen eingesetzt werden. Verfahren zur Selektivhydrierung sind beispielsweise in EP-A-0 827 944 beschrieben. Hydrierbare funktionelle Gruppen sind beispielsweise Nitrogruppen, Carbonylgruppen, Carboxylgruppen usw. Die Selektivhydrierung von Kohlenstoff-Kohlenstoff-Doppel- oder -Dreifachbindungen kann beispielsweise in Gegenwart von aromatischen Kernen erfolgen, wobei die aromatischen Kerne nicht hydriert werden.

Besonders bevorzugt wird der erfindungsgemäße monolithische Katalysator und der Reaktor zur Reinigung von Roh-Terephthalsäure durch katalytische hydrierende Nachbehandlung eingesetzt. Die Erfindung betrifft auch ein entsprechendes Reinigungsverfahren.

Als Katalysator für die hydrierende Nachbehandlung wird dabei insbesondere ein monolithischer Katalysator eingesetzt, der Palladium als Edelmetall auf den Kohlenstofffasern enthält. Der Anteil an Palladium beträgt bei Kohlenstoffgewebe vorzugsweise 10 bis 5000 mg Pd/m² Kohlenstoffgewebe.

Die Erfindung wird anhand der nachfolgenden Beispiele näher erläutert.

### Beispiele:

### Beispiel 1:

Die Herstellung eines monolithischen Pd-Kohle-Katalysators erfolgte mit 0,98 m² eines Kohlenstoff Fasergewebes von Tenax Fibers, die mit einer wässrigen Pd-Lösung mehrmals beschichtet wurde. Insgesamt wurden 910 mg Pd/m² Kohlenstoffgewebe aufgetragen. Es wurde dazu wie in EP-A-0 965 384 beschrieben vorgegangen. Man erhält ein Pd-Kohlenstoffgewebe mit folgenden Eigenschaften:
Spezifisches Gewicht: 92 g/m²
Pd-Gehalt: 1 %
Spezifische Oberfläche: 4,4 m²/g.

### Beispiel 2:

### Aktivitätstest, Hydrierung von Hydrodehydrolinanol (HDHL) zu Hydrolinanol (H-Lin)

Ein 0,2 m breites und 0,6 m langes Stück des Gewebes aus Beispiel 1 wurde lagenweise mit einem gewellten Edelstahl-Gewebe zu einem zylindrischen Monolithen verarbeitet, der zahlreiche parallel zur Längsachse des Zylinders durchströmbare Kanäle enthält. Die Wände dieser Kanäle bestehen aus dem edelinetallbeladenen Kohlenstoff Gewebe, das Edelstahl-Gewebe hat die Aufgabe, den Monolithen mechanisch zu stabilisieren.

Der erhaltene Monolith wurde in einen Reaktor eingebaut, in dem jeweils 0,5 kg reines HDHL ohne Lösungsmittel in einem diskontinuierlich betriebenen Kreislaufreaktor mit Kreisgas und Kreisflüssigkeit bei Querschnittsbelastungen von jeweils 200 m³/m²/h für die Gas- und Flüssigphase hydriert wurden.

### Mittels GC Analyse wurden folgende Hydrieraktivitäten ermittelt:

Der Versuch zeigte eine Hydriergeschwindigkeit von 17 % _{HDHL}/h, entsprechend einer RZA von 1,12 kg_{HDHL}/L_{Kat}/h. Das Produktgemisch blieb wasserklar.

| **Zeit** | **HDHL** | **H-Lin** | **THL** | **Rückstand** | **U/h** |
|---|---|---|---|---|---|
| 0 | 99,84 | 0,00 | 0,00 | 0,00 | |
| 30 | 93,26 | 5,95 | 0,23 | 0,00 | |
| 60 | 85,06 | 13,37 | 0,50 | 0,18 | 14,80 |
| 90 | 75,70 | 22,78 | 0,78 | 0,23 | |
| 120 | 67,45 | 30,23 | 1,10 | 0,71 | 16,22 |
| 150 | 57,32 | 39,96 | 1,39 | 0,92 | |
| 180 | 49,27 | 47,14 | 1,75 | 1,54 | 16,88 |

Nach dem Ausbau des Katalysators war der Monolith mechanisch einwandfrei erhalten.

### Beispiel 3:

### Hydrierung von 4-Carboxybenzaldehyd in Terephthalsäure Lösung; Pd-Verlust.

Eine Mischung von 54 g PTA in 146 g Wasser wurde bei 250°C für eine Woche unter Wasserstoff an einem gemäß Beispiel 2 hergestellten monolithischen Katalysator behandelt. Anschließend wurden sowohl die auskristallisierte Terephthalsäure-Fraktion als auch der wässrige Überstand auf Palladium analysiert.

Der Katalysator-Block war nach dem Ausbau mechanisch einwandfrei erhalten, es wurden keinerlei Abriebspuren im Reaktionsaustrag nachgewiesen. Ebenso konnten in beiden Fraktionen keine Pd-Spuren nachgewiesen werden.

### Beispiel 4:

In einem Druckautoklaven wurden 146 g Wasser und 54 g technische Roh-Terephthalsäure (2000 ppm 4-Carboxybenzaldehyd, Farbe: hellgelb) mit 8 g des gemäß Beispiel 1 hergestellten Pd/C-Gewebe zusammengegeben. Die Mischung wurde bei 270°C und 50 bar Druck unter Wasserstoff für 60 h gerührt. Das Produkt bestand aus weißen Terephthalsäurekristallen mit einem polarographisch bestimmten Gehalt an 4-Carboxybenzaldehyd von < 50 mg/kg.

## Patentansprüche

1. Verfahren zur Reinigung von Roh-Terephthalsäure durch katalytische hydrierende Nachbehandlung an einem Katalysatormaterial, das mindestens ein auf Kohlenstoffträger aufgebrachtes Hydriermetall enthält, **dadurch gekennzeichnet, dass** als Kohlenstoffträger Kohlenstofffasem eingesetzt werden.

2. Verfahren nach Anspruch 1, **dadurch gekennzeichnet, dass** im Katalysatormaterial die Kohlenstofffasem flächig in Form von Geweben, Gestricken, Gewirken und/oder Filzen oder in Form paralleler Fasern oder Bänder vorliegen.

3. Verfahren nach Anspruch 2, **dadurch gekennzeichnet, dass** das flächige Katalysatormaterial mindestens zwei einander gegenüberliegende Kanten aufweist, an denen das Katalysatormaterial in einem Reaktor formstabil befestigt ist.

4. Verfahren nach Anspruch 3, **dadurch gekennzeichnet, dass** sich mehrere Streifen des flächigen Katalysatonnaterials parallel zu einer Vorzugsrichtung im Reaktor erstrecken und so räumlich zueinander angeordnet sind, dass ein Abrieb an den Streifen durch gegenseitiges Berühren der Streifen untereinander oder mit den Reaktorwänden während des Reaktorbetriebes weitgehend verhindert wird.

5. Verfahren nach Anspruch 4, **dadurch gekennzeichnet, dass** die Streifen in dem Reaktor so angeordnet sind, dass ihre Vorzugsrichtung mit der Fließrichtung eines Reaktionsgemisches im wesentlichen übereinstimmt.

6. Verfahren nach Anspruch 1 oder 2, **dadurch gekennzeichnet, dass** als Katalysator ein monolithischer Katalysator eingesetzt wird, enthaltend das mindestens eine Katalysatormaterial, das das mindestens eine auf Kohlenstofffasern aufgebrachte Hydriermetall enthält, und mindestens ein vom Katalysatormaterial unterschiedliches und mit ihm verbundenes Stütz- oder Skelettelement, das das Katalysatormaterial mechanisch abstützt und in der monolithischen Form hält.

7. Verfahren nach Anspruch 6, **dadurch gekennzeichnet, dass** im monolithischen Katalysator das mindestens eine Stütz- oder Skelettelement aus Metall, Kunststoff oder Keramik aufgebaut ist.

8. Verfahren nach einem der Ansprüche 6 oder 7, **dadurch gekennzeichnet, dass** im monolithischen Katalysator das mindestens eine Stütz- oder Skelettelement flächig in Form von Geweben, Gestricken, Gewirken, Filzen und/oder Lochblechen vorliegt.

9. Verfahren nach Anspruch 8, **dadurch gekennzeichnet, dass** im monolithischen Katalysator die Stütz- oder Skelettelemente und das Katalysatormaterial als alternierende Schichten im Monolithen vorliegen.

10. Verfahren nach Anspruch 9, **dadurch gekennzeichnet, dass** im monolithischen Katalysator das Katalysatormaterial in Gewebeform und das mindestens eine Stützelement aus Metall in Gewebeform als flächige Schichtung vorliegen, die in einen zylindrischen Monolithen mit mehreren parallel zur Längsachse des Zylinders durchströmbaren Kanälen verformt ist.

11. Monolithischer Katalysator, enthaltend mindestens ein Katalysatormaterial, das mindestens ein auf Kohlenstofffasern aufgebrachtes Hydriermetall enthält, und mindestens ein vom Katalysatonnaterrial unterschiedliches und mit ihm verbundenes Stütz- oder Skelettelement, das das Katalysatormaterial mechanisch abstützt und in der monolithischen Form hält.

12. Monolithischer Katalysator nach Anspruch 11, wie er in einem der Ansprüche 7 bis 10 definiert ist.

13. Verfahren zur Herstellung eines monolithischen Katalysators nach Anspruch 11 oder 12 durch separate Herstellung des Katalysatormaterials und mindestens einen Stütz- oder Skelettelementes, Zusammenfügen beider und Verformen zu einem Monolithen.

14. Reaktor zur Durchführung eines Verfahrens gemäß einem der Ansprüche 1 bis 10, enthaltend ein flächiges Katalysatormaterial in Form von Geweben, Gestricken, Gewirken und/oder Filzen, das mindestens ein auf Kohlenstoffasern aufgebrachtes Hydriermetall enthält und mindestens zwei einander gegenüberliegende Kanten aufweist, an denen das Katalysatormaterial im Reaktor formstabil befestigt ist, oder enthaltend das Katalysatormaterial in Form paralleler Fasern oder Bänder.

15. Verwendung von Katalysatoren nach Anspruch 11 oder 12 oder Reaktoren nach Anspruch 14 zur Hydrierung von ungesättigten organischen Verbindungen.

16. Verfahren zur Selektivhydrierung von Kohlenstoff-Kohlenstoff-Doppel- oder - Dreifachbindungen und/oder hydrierbaren funktionellen Gruppen in diese enthaltenden organischen Verbindungen, **dadurch gekennzeichnet, dass** die Hydrierung an einem Katalysator gemäß Anspruch 11 oder 12 oder in einem Reaktor gemäß Anspruch 14 durchgeführt wird.

## Claims

1. A process for purifying crude terephthalic acid by catalytic hydrogenative after-treatment over a catalyst material comprising at least one hydrogenation metal applied to a carbon support, wherein carbon fibers are used as carbon support.

2. The process according to claim 1, wherein the carbon fibers in the catalyst material are present in sheet-like form as woven or knitted fabrics or meshes and/or felts or as parallel fibers or tapes.

3. The process according to claim 2, wherein the sheet-like catalyst material has at least two opposite edges at which the catalyst material is fastened in a reactor so as to retain its shape.

4. The process according to claim 3, wherein a plurality of strips of the sheet-like catalyst material extend parallel to a preferred direction in the reactor and they are arranged relative to one another in space in such a way that abrasion on the strips by contact of the strips with one another or with the reactor walls is largely prevented during operation of the reactor.

5. The process according to claim 4, wherein the strips are arranged in the reactor so that their preferred direction essentially coincides with the flow direction of a reaction mixture.

6. The process according to claim 1 or 2, wherein the catalyst used is a monolithic catalyst which comprises at least one catalyst material comprising at least one hydrogenation metal applied to carbon fibers and at least one support or skeletal element which is different from and joined to the catalyst material and supports the catalyst material mechanically and holds it in the monolithic form.

7. The process according to claim 6, wherein at least one support or skeletal element of metal, plastic or ceramic is built into the monolithic catalyst.

8. The process according to claim 6 or 7, wherein the support or skeletal element or elements is/are present in the monolithic catalyst in sheet-like form as woven or knitted fabrics/meshes, felts and/or perforated sheets.

9. The process according to claim 8, wherein the support or skeletal elements and the catalyst material are present as alternating layers in the monolithic catalyst.

10. The process according to claim 9, wherein the catalyst material is present in the monolithic catalyst in woven form and the support element or elements is/are present in the form of a metal weave as sheet-like layer which is shaped to form a cylindrical monolith having a plurality of flow channels parallel to the longitudinal axis of the cylinder.

11. A monolithic catalyst which comprises at least one catalyst material comprising at least one hydrogenation metal applied to carbon fibers and at least one support or skeletal element which is different from and joined to the catalyst material and supports the catalyst material mechanically and holds it in the monolithic form.

12. The monolithic catalyst according to as defined in any of claims 7 to 10.

13. A process for producing a monolithic catalyst according to claim 11 or 12 by separately producing the catalyst material and at least one support or skeletal element, combining the two and shaping them to form a monolith.

14. A reactor for carrying out a process according to any of claims 1 to 10, comprising a sheet-like catalyst material in the form of woven or knitted fabrics/meshes and/or felts which comprises at least one hydrogenation metal applied to carbon fibers and has at least two opposite edges at which the catalyst material is fastened in the reactor so as to retain its shape, or comprising the catalyst material as parallel fibers or tapes.

15. The use of the catalyst according to claim 11 or 12 or the reactor according to claim 14 for the hydrogenation of unsaturated organic compounds.

16. A process for the selective hydrogenation of carbon-carbon double or triple bonds and/or hydrogenatable functional groups in organic compounds in which these are present, which comprises carrying out the hydrogenation over a catalyst according to claim 11 or 12 or in a reactor according to claim 14.

## Revendications

1. Procédé de purification d'acide téréphtalique brut par un traitement secondaire d'hydrogénation catalytique sur une matière catalytique, qui contient au moins un métal d'hydrogénation appliqué sur un support carboné, **caractérisé en ce que**, comme support carboné, on met en oeuvre des fibres de carbone.

2. Procédé suivant la revendication 1, **caractérisé en ce que**, dans la matière catalytique, les fibres de carbone se présentent à plat sous forme de tissus, de tricots, de tissus à mailles et/ou de feutres ou sous la forme de fibres ou bandes parallèles.

3. Procédé suivant la revendication 2, **caractérisé en ce que** la matière catalytique plane présente au moins deux bords opposés au niveau desquels la matière catalytique est fixée de manière à présenter une forme stable dans un réacteur.

4. Procédé suivant la revendication 3, **caractérisé en ce que** plusieurs rubans de la matière catalytique plane s'étendent parallèlement à une orientation préférentielle dans le réacteur et sont agencés spatialement l'un par rapport à l'autre de façon à ce qu'une usure des rubans par contact mutuel des rubans entre eux ou avec les parois du réacteur pendant le fonctionnement du réacteur soit largement empêchée.

5. Procédé suivant la revendication 4, **caractérisé en ce que** les rubans sont agencés dans le réacteur de façon à ce que leur orientation préférentielle corresponde sensiblement au sens d'écoulement d'un mélange réactionnel.

6. Procédé suivant la revendication 1 ou 2, **caractérisé en ce que**, comme catalyseur, on met en oeuvre un catalyseur monolithique contenant ladite au moins une matière catalytique, qui contient ledit au moins un métal d'hydrogénation appliqué sur des fibres de carbone et au moins un élément de support ou de squelette différent de la matière catalytique et relié à elle, qui soutient mécaniquement la matière catalytique et la maintient dans la forme monolithique.

7. Procédé suivant la revendication 6, **caractérisé en ce que**, dans le catalyseur monolithique, ledit au moins un élément de soutien ou de squelette est constitué de métal, de substance synthétique ou de céramique.

8. Procédé suivant l'une des revendications 6 ou 7, **caractérisé en ce que**, dans le catalyseur monolithique, ledit au moins un élément de soutien ou de squelette se présente à plat sous la forme de tissus, de tricots, de tissus à mailles, de feutres et/ou de plaques perforées.

9. Procédé suivant la revendication 8, **caractérisé en ce que**, dans le catalyseur monolithique, les éléments de soutien ou de squelette et la matière catalytique se présentent sous la forme de couches alternées dans le monolithe.

10. Procédé suivant la revendication 9, **caractérisé en ce que**, dans le catalyseur monolithique, la matière catalytique sous forme de tissu et ledit au moins un élément de soutien en métal sous forme de tissu se présentent sous la forme d'une stratification aplatie qui est mise en forme en un monolithe cylindrique avec plusieurs canaux qui peuvent être traversés parallèlement à l'axe longitudinal du cylindre.

11. Catalyseur monolithique, contenant au moins une matière catalytique, qui contient au moins un métal d'hydrogénation appliqué sur des fibres de carbone et au moins un élément de soutien ou de squelette différent de la matière catalytique et relié à elle, qui soutient mécaniquement la matière catalytique et la maintient dans la forme monolithique.

12. Catalyseur monolithique suivant la revendication 11, tel que défini dans une des revendications 7 à 10.

13. Procédé de préparation d'un catalyseur monolithique suivant la revendication 11 ou 12, par préparation séparée de la matière catalytique et d'au moins un élément de soutien ou de squelette, assemblage des deux et formage en un monolithe.

14. Réacteur pour la mise en oeuvre d'un procédé suivant l'une des revendications 1 à 10, contenant une matière catalytique plane sous forme de tissus, de tricots, de tissus à mailles et/ou de feutres, qui contient au moins un métal d'hydrogénation appliqué sur des fibres de carbone et présente au moins deux bords opposés au niveau desquels la matière catalytique est fixée de manière à présenter une forme stable dans le réacteur, ou contenant la matière catalytique sous forme de fibres ou bandes parallèles.

15. Utilisation de catalyseurs suivant la revendication 11 ou 12 ou de réacteurs suivant la revendication 14, pour l'hydrogénation de composés organiques insaturés.

16. Procédé d'hydrogénation sélective de doubles ou triples liaisons carbone-carbone et/ou de groupes fonctionnels hydrogénables dans des composés organiques les contenant, **caractérisé en ce que** l'hydrogénation est effectuée sur un catalyseur suivant la revendication 11 ou 12 ou dans un réacteur suivant la revendication 14.
